# EUROPEAN PATENT APPLICATION

(11) **EP 3 871 597 A1**
(43) Date of publication of application: **01.09.2021**
(21) Application number: 20160208.3
(22) Date of filing: 28.02.2020
(51) Int. Cl.: A61B 5/08, G01N 33/497

(54) **DIAGNOSIS OF HEART FAILURE**

(71) Applicant: Universitair Ziekenhuis Antwerpen, 2650 Edegem (BE); Universiteit Antwerpen, 2000 Antwerpen (BE); Vlaamse Instelling voor Technologisch Onderzoek (VITO), 2400 Mol (BE)
(72) Inventor: Claeys, Marc, 2070 Zwijndrecht (BE); Jorens, Philippe, 2630 Aartselaar (BE); Koppen, Gudrun, 2440 Geel (BE)
(74) Representative: De Clercq & Partners

(57) **Abstract**

The present invention relates to differential diagnosis of acute heart failure and pulmonary diseases, such as chronic obstructive pulmonary disorder or community acquired pneumonia, in particular in subjects having dyspnea. Discrimination between acute heart failure and pulmonary diseases is achieved based on acetone and/or isoprene concentration in exhaled breath.

## Description

### FIELD OF THE INVENTION

The present invention relates to the field of medical diagnosis. The present invention in particular relates to diagnosis of heart failure, such as acute heart failure.

### BACKGROUND OF THE INVENTION

One of the main reasons for admission to the Emergency Department (ED) is acute dyspnea, often caused by Acute Heart Failure (AHF). However, patients that present with dyspnea (breathlessness, air hunger or a feeling of suffocation) may be either suffering from cardiac or pulmonary diseases, making it difficult to immediately discriminate them. A delayed or erroneous diagnosis of AHF has major consequences, as it is associated with increased mortality. In addition, it increases the risk of intensive care unit admission and prolongs hospital stay, all associated with increased healthcare costs. Therefore a rapid and accurate diagnostic work-up for dyspnea and AHF is critical, especially in elderly patients with multiple comorbidities. The European Society of Cardiology (ESC) guidelines for heart failure recommend a work-up that includes the patient's history, physical examination, chest radiography (CXR) and 12-lead electrocardiography in patients with suspected AHF. However, this approach is often imprecise, leading to uncertain diagnosis in even up to 44% of patients. The addition of the biomarker Brain Natriuretic Peptide (BNP) or N-Terminal pro BNP (NT-proBNP) level, recommended by the ESC, has improved the diagnostic accuracy, but the misclassification rate still remains unacceptably high. More recently, a randomized control trial found that the implementation of lung ultrasound (LUS) further improved this diagnostic accuracy. However, its use in the acutely dyspneic patient can be limited due to availability of a skilled practitioner. Therefore, there is a need for new, point-of-care diagnostic tools to help discriminate AHF from pulmonary causes of dyspnea such as Chronic Obstructive Pulmonary Disease (COPD) or Community Acquired Pneumonia (CAP).

### SUMMARY OF THE INVENTION

The present inventors have surprisingly found that breath acetone and/or isoprene concentration can be used as a diagnostic marker to discriminate between heart failure, such as acute heart failure, and pulmonary diseases, such as chronic obstructive pulmonary disease (COPD) or community acquired pneumonia (CAP). As such, breath acetone and/or isoprene concentration can therefore be used both to diagnose heart failure and pulmonary diseases. The inventors have further found that such diagnosis, and in particular differential diagnosis can be made in subjects having dyspnea. Accordingly, breath acetone and/or isoprene concentration can therefore be used both to diagnose the underlying cause of dyspnea, such as heart failure and pulmonary diseases.

In an aspect, the invention relates to a method for discriminating between acute heart failure and a pulmonary disease in a subject having dyspnea, comprising determining acetone and/or isoprene concentration in a breath sample from said subject. In an aspect, the invention relates to a method for discriminating between acute heart failure and a pulmonary disease in a subject having dyspnea, comprising determining acetone concentration in a breath sample from said subject. In an aspect, the invention relates to a method for discriminating between acute heart failure and a pulmonary disease in a subject having dyspnea, comprising determining isoprene concentration in a breath sample from said subject. In an aspect, the invention relates to a method for discriminating between acute heart failure and a pulmonary disease in a subject having dyspnea, comprising determining acetone and isoprene concentration in a breath sample from said subject.

In another aspect, the invention relates to the use of acetone and/or isoprene concentration in a breath sample from a subject having dyspnea as a marker for discriminating between acute heart failure and a pulmonary disease in said subject. In another aspect, the invention relates to the use of acetone concentration in a breath sample from a subject having dyspnea as a marker for discriminating between acute heart failure and a pulmonary disease in said subject. In another aspect, the invention relates to the use of isoprene concentration in a breath sample from a subject having dyspnea as a marker for discriminating between acute heart failure and a pulmonary disease in said subject. In another aspect, the invention relates to the use of acetone and isoprene concentration in a breath sample from a subject having dyspnea as a marker for discriminating between acute heart failure and a pulmonary disease in said subject.
In certain embodiments, the pulmonary disease is chronic obstructive pulmonary disorder (COPD).

In certain embodiments, the pulmonary disease is (non heart failure related) pneumonia. In certain embodiments, the pulmonary disease is community acquired pneumonia (CAP).

In certain embodiments, acute heart failure is diagnosed in said subject if the acetone concentration is at least 700 to 900 ppbV, preferably ranging from between 700 and 900 ppbV to 10000 ppbV.

In certain embodiments, acute heart failure is diagnosed in said subject if the acetone concentration is at least 864 ppbV, preferably ranging from 864 ppbV to 10000 ppbV.

In certain embodiments, a pulmonary disease is diagnosed in said subject if the acetone concentration is less than 700 to 900 ppbV, preferably ranging from between 300 ppbV and 700 to 900 ppbV.

In certain embodiments, a pulmonary disease is diagnosed in said subject if the acetone concentration is less than 864 ppbV, preferably ranging from between 300 ppbV and less than 864 ppbV.

In certain embodiments, acute heart failure is diagnosed in said subject if the isoprene concentration is at least 25 to 35 ppbV, preferably ranging from between 25 and 35 ppbV to 200 ppbV.

In certain embodiments, acute heart failure is diagnosed in said subject if the isoprene concentration is at least 30 ppbV, preferably ranging from 30 ppbV to 200 ppbV.

In certain embodiments, a pulmonary disease is diagnosed in said subject if the isoprene concentration is less than 25 to 35 ppbV, preferably ranging from between 10 ppbV and 25 to 35 ppbV.

In certain embodiments, a pulmonary disease is diagnosed in said subject if the isoprene concentration is less than 30 ppbV, preferably ranging from between 10 ppbV and less than 30 ppbV.

In certain embodiments, the subject has left ventricular dysfunction, in particular a decreased ejection fraction and/or wherein said subject has right ventricular dysfunction, in particular a decreased ejection fraction. In certain embodiments, the invention relates to a method for discriminating between acute heart failure associated with left ventricular dysfunction, in particular a decreased ejection fraction and/or right ventricular dysfunction, in particular a decreased ejection fraction and a pulmonary disease in a subject having dyspnea, comprising determining acetone and/or isoprene concentration in a breath sample from said subject.

In certain embodiments, the subject has a mean left and/or right ejection fraction of less than 50%, preferably less than 40%, more preferably less than 30%. In certain embodiments, the invention relates to a method for discriminating between acute heart failure associated a mean left and/or right ejection fraction of less than 50%, preferably less than 40%, more preferably less than 30% and a pulmonary disease in a subject having dyspnea, comprising determining acetone and/or isoprene concentration in a breath sample from said subject.

In certain embodiments, the subject is admitted to a hospital emergency department.

The invention is in particular captured by the appended claims, which are incorporated herein explicitly by reference.

### FIGURES

**Figure 1****:** Breath sample volatile organic compound (VOC) analysis according to an embodiment of the invention.

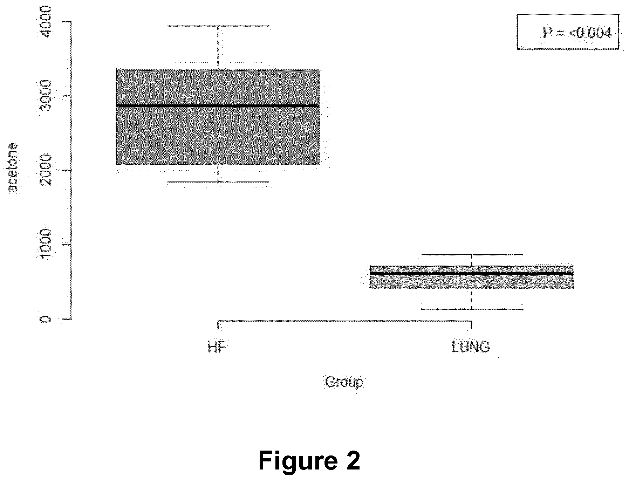

**Figure 2****:** Boxplot graph of breath acetone concentration (expressed in parts per billion volume fraction, ppbV) in subjects having acute heart failure ("HF") and pulmonary disease ("LUNG").

### DETAILED DESCRIPTION OF THE INVENTION

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of", as well as the terms "consisting essentially of", "consists essentially" and "consists essentially of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-20% or less, preferably +/-10% or less, more preferably +/-5% or less, and still more preferably +/-1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the following detailed description of the invention, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

Preferred statements (aspects, features) and embodiments of this invention are set herein below. Each statement and embodiment of the invention so defined may be combined with any other statement and/or embodiment unless clearly indicated to the contrary. In particular, any feature indicated as being preferred or advantageous may be combined with any other feature or features or statements indicated as being preferred or advantageous. Hereto, the present invention is in particular captured by any one or any combination of one or more of the below numbered aspects and embodiments (statements) 1 to 81.
1. A method for diagnosing heart failure, such as acute heart failure, comprising determining acetone and/or isoprene concentration in a breath sample from a subject.
2. A method for diagnosing a pulmonary disease, such as COPD or pneumonia, in particular CAP, comprising determining acetone and/or isoprene concentration in a breath sample from a subject.
3. A method for discriminating between heart failure, such as acute heart failure, and a pulmonary disease, such as COPD or pneumonia, in particular CAP, comprising determining acetone and/or isoprene concentration in a breath sample from a subject.
4. A method for differentially diagnosing heart failure, such as acute heart failure, or a pulmonary disease, such as COPD or pneumonia, in particular CAP, in a subject, comprising determining acetone and/or isoprene concentration in a breath sample from said subject.
5. A method for diagnosing dyspnea as being caused by heart failure, such as acute heart failure, comprising determining acetone and/or isoprene concentration in a breath sample from said subject.
6. A method for diagnosing dyspnea as being caused by a pulmonary disease, such as COPD or pneumonia, in particular CAP, comprising determining acetone and/or isoprene concentration in a breath sample from said subject.
7. A method for discriminating between dyspnea caused by heart failure, such as acute heart failure, or a pulmonary disease, such as COPD or pneumonia, in particular CAP, comprising determining acetone and/or isoprene concentration in a breath sample from said subject.
8. A method for differentially diagnosing dyspnea as being caused by heart failure, such as acute heart failure, or a pulmonary disease, such as COPD or pneumonia, in particular CAP, comprising determining acetone and/or isoprene concentration in a breath sample from said subject.
9. A method for excluding a diagnosis of heart failure, such as acute heart failure, comprising determining acetone and/or isoprene concentration in a breath sample from said subject.
10. A method for excluding a diagnosis of a pulmonary disease, such as COPD or pneumonia, in particular CAP, comprising determining acetone and/or isoprene concentration in a breath sample from said subject.
11. A method for excluding a diagnosis of dyspnea as being caused by heart failure, such as acute heart failure, comprising determining acetone and/or isoprene concentration in a breath sample from said subject.
12. A method for excluding a diagnosis of dyspnea as being caused by a pulmonary disease, such as COPD or pneumonia, in particular CAP, comprising determining acetone and/or isoprene concentration in a breath sample from said subject.
13. Use of acetone and/or isoprene concentration in a breath sample from a subject as a marker for diagnosing heart failure, such as acute heart failure.
14. Use of acetone and/or isoprene concentration in a breath sample from a subject as a marker for diagnosing a pulmonary disease, such as COPD or pneumonia, in particular CAP, comprising determining acetone concentration in a breath sample from a subject.
15. Use of acetone and/or isoprene concentration in a breath sample from a subject as a marker for discriminating between heart failure, such as acute heart failure, and a pulmonary disease, such as COPD or pneumonia, in particular CAP.
16. Use of acetone and/or isoprene concentration in a breath sample from a subject as a marker for differentially diagnosing heart failure, such as acute heart failure, or a pulmonary disease, such as COPD or pneumonia, in particular CAP, in a subject.
17. Use of acetone and/or isoprene concentration in a breath sample from a subject as a marker for diagnosing dyspnea as being caused by heart failure, such as acute heart failure.
18. Use of acetone and/or isoprene concentration in a breath sample from a subject as a marker for diagnosing dyspnea as being caused by a pulmonary disease, such as COPD or pneumonia, in particular CAP.
19. Use of acetone and/or isoprene concentration in a breath sample from a subject as a marker for discriminating between dyspnea caused by heart failure, such as acute heart failure, or a pulmonary disease, such as COPD or pneumonia, in particular CAP.
20. Use of acetone and/or isoprene concentration in a breath sample from a subject as a marker for differentially diagnosing dyspnea as being caused by heart failure, such as acute heart failure, or a pulmonary disease, such as COPD or pneumonia, in particular CAP.
21. Use of acetone and/or isoprene concentration in a breath sample from a subject as a marker for excluding a diagnosis of heart failure, such as acute heart failure.
22. Use of acetone and/or isoprene concentration in a breath sample from a subject as a marker for excluding a diagnosis of a pulmonary disease, such as COPD or pneumonia, in particular CAP.
23. Use of acetone and/or isoprene concentration in a breath sample from a subject as a marker for excluding a diagnosis of dyspnea as being caused by heart failure, such as acute heart failure.
24. Use of acetone and/or isoprene concentration in a breath sample from a subject as a marker for excluding a diagnosis of dyspnea as being caused by a pulmonary disease, such as COPD or pneumonia, in particular CAP.
25. The method or use according to any of statements 1 to 24, wherein said heart failure is acute heart failure.
26. The method or use according to any of statements 1 to 25, wherein said heart failure is decompensated heart failure.
27. The method or use according to any of statements 1 to 26, wherein said heart failure is heart failure with congestion.
28. The method or use according to any of statements 1 to 26, wherein said heart failure is heart failure without congestion.
29. The method or use according to any of statements 1 to 28, wherein said heart failure is not chronic heart failure.
30. The method or use according to any of statements 1 to 29, wherein said subject has left ventricular dysfunction or wherein said heart failure is associated with or caused by left ventricular dysfunction.
31. The method or use according to any of statements 1 to 30, wherein said subject has right ventricular dysfunction or wherein said heart failure is associated with or caused by right ventricular dysfunction.
32. The method or use according to any of statements 1 to 31, wherein said subject has a decreased ejection fraction or wherein said heart failure is associated with or caused by a decreased ejection fraction.
33. The method or use according to any of statements 1 to 32, wherein said subject has a mean left ejection fraction of less than 50%, preferably less than 40%, more preferably less than 30% or wherein said heart failure is associated with or caused by a mean left ejection fraction of less than 50%, preferably less than 40%, more preferably less than 30%.
34. The method or use according to any of statements 1 to 33, wherein said subject has a mean right ejection fraction of less than 50%, preferably less than 40%, more preferably less than 30% or wherein said heart failure is associated with or caused by a mean right ejection fraction of less than 50%, preferably less than 40%, more preferably less than 30%.
35. The method or use according to any of statements 1 to 34, wherein said subject has cardiomyopathy or wherein said heart failure is associated with or caused by cardiomyopathy.
36. The method or use according to any of statements 1 to 35, wherein said subject has dilated, hypertrophic, or restrictive cardiomyopathy or wherein said heart failure is associated with or caused by dilated, hypertrophic, or restrictive cardiomyopathy.
37. The method or use according to any of statements 1 to 36, wherein said subject has pulmonary edema or pulmonary congestion or wherein said heart failure is associated with or caused by pulmonary edema or pulmonary congestion.
38. The method or use according to any of statements 1 to 37, wherein said pulmonary disease is a respiratory disease.
39. The method according to any of statements 1 to 38, wherein said pulmonary disease is an infection, such as a viral, bacterial, or fungal infection.
40. The method according to any of statements 1 to 38, wherein said pulmonary disease is not an infection.
41. The method or use according to any of statements 1 to 40, wherein said pulmonary disease is selected from chronic obstructive pulmonary disorder, (non-heart failure related) pneumonia such as community acquired pneumonia, asthma, lung cancer, (chronic) bronchitis, emphysema, cystic fibrosis, or (non-heart failure related) pleural effusion.
42. The method or use according to any of statements 1 to 41, wherein said pulmonary disease is chronic obstructive pulmonary disorder (COPD).
43. The method according to any of statements 1 to 41, wherein said pulmonary disease is pneumonia.
44. The method according to any of statements 1 to 41, wherein said pulmonary disease is community acquired pneumonia.
45. The method or use according to any of statements 1 to 44, wherein said subject has dyspnea.
46. The method or use according to any of statements 1 to 46, wherein said subject has self-diagnosed dyspnea.
47. The method or use according to any of statements 1 to 46, wherein said subject has clinically validated or diagnosed dyspnea.
48. The method or use according to any of statements 1 to 47, wherein said subject is admitted to a hospital emergency department.
49. The method or use according to any of statements 1 to 48, wherein said subject does not have diabetes, in particular type 1 diabetes.
50. The method or use according to any of statements 1 to 49, wherein said subject is diagnosed with heart failure based on a predetermined acetone and/or isoprene threshold concentration in said breath sample.
51. The method or use according to any of statements 1 to 49, wherein said subject is diagnosed with a pulmonary disease based on a predetermined acetone and/or isoprene threshold concentration in said breath sample.
52. The method or use according to any of statements 1 to 49, wherein said subject is (differentially) diagnosed with heart failure or a pulmonary disease based on a predetermined acetone and/or isoprene threshold concentration in said breath sample.
53. The method or use according claim 52, wherein said subject is diagnosed with heart failure if the acetone and/or isoprene concentration in said breath sample is at least the acetone and/or isoprene threshold concentration, and wherein said subject is diagnosed as not having heart failure if the acetone and/or isoprene concentration in said breath sample is below the acetone and/or isoprene threshold concentration.
54. The method or use according claim 52, wherein said subject is diagnosed with a pulmonary disease if the acetone and/or isoprene concentration in said breath sample is below the acetone and/or isoprene threshold concentration, and wherein said subject is diagnosed as not having a pulmonary disease if the acetone and/or isoprene concentration in said breath sample is at least the acetone and/or isoprene threshold concentration.
55. The method or use according claim 52, wherein said subject is diagnosed with heart failure if the acetone and/or isoprene concentration in said breath sample is at least the acetone and/or isoprene threshold concentration, and wherein said subject is diagnosed with a pulmonary disease if the acetone and/or isoprene concentration in said breath sample is below the acetone and/or isoprene threshold concentration.
56. The method or use according to any of statements 1 to 55, wherein acute heart failure is diagnosed in said subject if the acetone concentration is at least 700 to 900 ppbV.
57. The method or use according to any of statements 1 to 56, wherein acute heart failure is diagnosed in said subject if the acetone concentration is ranging from between (and including) 700 to 900 ppbV and 10000 ppbV.
58. The method or use according to any of statements 1 to 57, wherein acute heart failure is diagnosed in said subject if the acetone concentration is at least 864 ppbV.
59. The method or use according to any of statements 1 to 58, wherein acute heart failure is diagnosed in said subject if the acetone concentration is ranging from between (and including) 864 ppbV and 10000 ppbV.
60. The method or use according to any of statements 1 to 59, wherein a pulmonary disease is diagnosed in said subject if the acetone concentration is less than 700 to 900 ppbV.
61. The method or use according to any of statements 1 to 60, wherein a pulmonary disease is diagnosed in said subject if the acetone concentration is ranging from between 300 ppbV and 700 to 900 ppbV.
62. The method or use according to any of statements 1 to 61, wherein a pulmonary disease is diagnosed in said subject if the acetone concentration is less than 864 ppbV.
63. The method or use according to any of statements 1 to 62, wherein a pulmonary disease is diagnosed in said subject if the acetone concentration is ranging from between 300 ppbV and 864 ppbV.
64. The method or use according to any of statements 1 to 63, wherein acute heart failure is diagnosed in said subject if the isoprene concentration is at least 25 to 35 ppbV.
65. The method or use according to any of statements 1 to 64, wherein acute heart failure is diagnosed in said subject if the isoprene concentration is ranging from between (and including) 25 to 35 ppbV and 200 ppbV.
66. The method or use according to any of statements 1 to 65, wherein acute heart failure is diagnosed in said subject if the isoprene concentration is at least 30 ppbV.
67. The method or use according to any of statements 1 to 66, wherein acute heart failure is diagnosed in said subject if the isoprene concentration is ranging from between (and including) 30 ppbV and 200 ppbV.
68. The method or use according to any of statements 1 to 67, wherein a pulmonary disease is diagnosed in said subject if the isoprene concentration is less than 25 to 35 ppbV.
69. The method or use according to any of statements 1 to 68, wherein a pulmonary disease is diagnosed in said subject if the isoprene concentration is ranging from between 10 ppbV and 25 to 35 ppbV.
70. The method or use according to any of statements 1 to 69, wherein a pulmonary disease is diagnosed in said subject if the isoprene concentration is less than 30 ppbV.
71. The method or use according to any of statements 1 to 70, wherein a pulmonary disease is diagnosed in said subject if the isoprene concentration is ranging from between 10 ppbV and 35 ppbV.
72. The method or use according to any of statements 56 to 71, wherein said acetone and/or isoprene concentration is as determined by gas chromatography and/or mass spectrometry.
73. The method or use according to statement 72, wherein said mass spectrometry is ion flow tube mass spectrometry.
74. The metrhod according to any of statements 1 to 73, wherein said subject is at least 55 years old, preferably at least 60 years old.
75. The method or use according to any of statements 1 to 74, further comprising determining isoprene and/or pentane concentration in a breath sample from said subject.
76. The method or use according to any of statements 1 to 75, further comprising treating said subject.
78. The method or use according to any of statements 1 to 76, further comprising treating said subject for said heart failure.
78. The method or use according to any of statements 1 to 76, further comprising treating said subject for said pulmonary disease.
79. The method or use according to any of statements 1 to 78, wherein (only) acetone concentration is determined or wherein (only) acetone is a marker.
80. The method or use according to any of statements 1 to 78, wherein (only) isoprene concentration is determined or wherein (only) isoprene is a marker.
81. The method or use according to any of statements 1 to 78, wherein acetone and isoprene concentrations are determined or wherein acetone and isoprene are markers.

Statements 1 to 24 are all interrelated and may be considered in certain embodiments as alternative approaches or expressions of the present invention.

The present invention relates to the diagnosis of heart failure, such as preferably acute heart failure, and/or pulmonary disease, such as preferably chronic obstructive pulmonary disorder or (community acquired) pneumonia, and in particular to discriminating between heart failure and pulmonary disease in order to differentially diagnose heart failure and pulmonary disease. Diagnosis according to the invention is based on determining acetone and/or isoprene concentration in exhaled breath samples, and is hence a non-invasive diagnosis. Predetermined breath acetone and/or isoprene concentration thresholds allow diagnosis of heart failure and pulmonary disease and in particular allow discrimination between heart failure and pulmonary disease. Preferably, subjects to be diagnosed have dyspnea, such as subjects admitted to the emergency department of a hospital and having dyspnea. In preferred embodiments acetone concentration is determined.

As used herein, the term "heart failure" corresponds to its ordinary meaning known in the art. By means of further guidance, and without limitation, heart failure relates to a condition when the heart is unable to pump sufficiently to maintain blood flow to meet the body's needs. Signs and symptoms of heart failure may include shortness of breath, excessive tiredness, and systemic and/or pulmonary edema. Common causes of heart failure include coronary artery disease, including a previous myocardial infarction (heart attack), high blood pressure, atrial fibrillation, valvular heart disease, excess alcohol use, infection, and cardiomyopathy. These cause heart failure by changing either the structure or the function of the heart. The left side of the heart receives oxygen-rich blood from the lungs and pumps it forward to the systemic circulation (the rest of the body except for the pulmonary circulation). Failure of the left side of the heart causes blood to back up (be congested) into the lungs, causing respiratory symptoms as well as fatigue due to insufficient supply of oxygenated blood. The two types of left ventricular heart failure - heart failure with reduced ejection fraction (HFrEF), and heart failure with preserved ejection fraction (HFpEF) - are based on whether the ability of the left ventricle to contract, or to relax, is affected. Backward failure of the left ventricle causes congestion of the lungs' blood vessels, and so the symptoms are predominantly respiratory in nature. Backward failure can be subdivided into the failure of the left atrium, the left ventricle or both within the left circuit. The person will have dyspnea (shortness of breath) on exertion and in severe cases, dyspnea at rest. Right-sided heart failure is often caused by pulmonary heart disease (cor pulmonale), which is typically caused by difficulties of the pulmonary circulation, such as pulmonary hypertension or pulmonic stenosis. Backward failure of the right ventricle leads to congestion of systemic capillaries. This generates excess fluid accumulation in the body. This causes swelling under the skin (termed peripheral edema or anasarca) and usually affects the dependent parts of the body first (causing foot and ankle swelling in people who are standing up, and sacral edema in people who are predominantly lying down). Treatment depends on the severity and cause of the disease. In people with chronic stable mild heart failure, treatment commonly consists of lifestyle modifications such as stopping smoking, physical exercise, and dietary changes, as well as medications. In those with heart failure due to left ventricular dysfunction, angiotensin converting enzyme inhibitors, angiotensin receptor blockers, or valsartan/sacubitril along with beta blockers are recommended. For those with severe disease, aldosterone antagonists, or hydralazine with a nitrate may be used. Diuretics are useful for preventing fluid retention and the resulting shortness of breath. Sometimes, depending on the cause, an implanted device such as a pacemaker or an implantable cardiac defibrillator (ICD) may be recommended. In some moderate or severe cases, cardiac resynchronization therapy (CRT) or cardiac contractility modulation may be of benefit. A ventricular assist device (for the left, right, or both ventricles), or occasionally a heart transplant may be recommended in those with severe disease that persists despite all other measures. Acute (decompensated) heart failure (ADHF) is a sudden worsening of the signs and symptoms of heart failure, which typically includes difficulty breathing (dyspnea), leg or feet swelling, and fatigue. ADHF is a common and potentially serious cause of acute respiratory distress. The condition is caused by severe congestion of multiple organs by fluid that is inadequately circulated by the failing heart. An attack of decompensation can be caused by underlying medical illness, such as myocardial infarction, an abnormal heart rhythm, infection, or thyroid disease. In acute (decompensated) heart failure, the immediate goal is to re-establish adequate perfusion and oxygen delivery to end organs. This entails ensuring that airway, breathing, and circulation are adequate. Management consists of propping up the head of the patient, giving oxygen to correct hypoxemia, administering morphine, diuretics like furosemide, addition of an ACE inhibitor, use of nitrates and use of digoxin if indicated for the heart failure and if arrhythmic.

In certain embodiments, heart failure is acute heart failure. In certain embodiments, heart failure is decompensated heart failure. In certain embodiments, heart failure is acute decompensated heart failure. In certain embodiments, acute heart failure and decompensated heart failure are used interchangeably herein. Acute heart failure may be an acute exacerbation of chronic heart failure or may be "new" acute heart failure in the sense that no previous diagnosis or symptoms of heart failure have been established and may for instance be triggered by clinical situations such as acute myocardial infarction or hypertensive crisis.

As used herein "pulmonary disease" refers to a disease affecting the lungs or airways, and which may affect lung or airway functionality and/or structure. As such, a pulmonary disease may include respiratory diseases. By means of example, and without limitation, pulmonary diseases include chronic obstructive pulmonary disorder (COPD), pneumonia, including community acquired pneumonia (CAP), asthma, lung cancer, cystic fibrosis, pleural effusion (in particular not caused by heart failure), emphysema, asthma, and bronchitis, in particular chronic bronchitis. In preferred embodiments, the pulmonary disease is COPD or pneumonia, in particular community acquired pneumonia (CAP). In a preferred embodiment, the pulmonary disease is COPD. In a preferred embodiment, the pulmonary disease is pneumonia. In a preferred embodiment, the pulmonary disease is CAP.

The term "chronic obstructive pulmonary disease" or "COPD" has its ordinary meaning known in the art. By means of further guidance, and without limitation, chronic obstructive pulmonary disease (COPD) is a type of obstructive lung disease characterized by long-term breathing problems and poor airflow. Chronic obstructive pulmonary disease (COPD) is a chronic inflammatory lung disease that causes obstructed airflow from the lungs. Symptoms include breathing difficulty, cough, mucus (sputum) production and wheezing. It's caused by long-term exposure to irritating gases or particulate matter, most often from cigarette smoke. People with COPD are at increased risk of developing heart disease, lung cancer and a variety of other conditions. Emphysema and chronic bronchitis are the two most common conditions that contribute to COPD. Chronic bronchitis and emphysema are in fact older terms used for different types of COPD. The term "chronic bronchitis" is still used to define a productive cough that is present for at least three months each year for two years. Those with such a cough are at a greater risk of developing COPD. Chronic bronchitis is inflammation of the lining of the bronchial tubes, which carry air to and from the air sacs (alveoli) of the lungs. It's characterized by daily cough and mucus (sputum) production. The term "emphysema" is also used for the abnormal presence of air or other gas within tissues. Emphysema is a condition in which the alveoli at the end of the smallest air passages (bronchioles) of the lungs are destroyed as a result of damaging exposure to cigarette smoke and other irritating gases and particulate matter. COPD is a progressive disease, meaning it typically worsens over time. Eventually, everyday activities such as walking or getting dressed become difficult. Shortness of breath (dyspnea) is a common symptom and is often the most distressing. Typically, the shortness of breath is worse on exertion of a prolonged duration and worsens over time. In the advanced stages, or end stage pulmonary disease, it occurs during rest and may be always present. An acute exacerbation of COPD is defined as increased shortness of breath, increased sputum production, a change in the color of the sputum from clear to green or yellow, or an increase in cough in someone with COPD.

As used herein, "pneumonia" has its ordinary meaning known in the art. By means of further guidance, and without limitation, pneumonia is an inflammatory condition of the lung affecting primarily the small air sacs known as alveoli. Typically symptoms include some combination of productive or dry cough, chest pain, fever, and trouble breathing. Pneumonia is usually caused by infection with viruses or bacteria and less commonly by other microorganisms, certain medications and conditions such as autoimmune diseases. Risk factors include cystic fibrosis, chronic obstructive pulmonary disease (COPD), asthma, diabetes, heart failure, a history of smoking, a poor ability to cough such as following a stroke, and a weak immune system. Diagnosis is often based on the symptoms and physical examination. Chest X-ray, blood tests, and culture of the sputum may help confirm the diagnosis. The disease may be classified by where it was acquired with community, hospital, or health care associated pneumonia. Community-acquired pneumonia refers to pneumonia (any of several lung diseases) contracted by a person with little contact with the healthcare system. The chief difference between hospital-acquired pneumonia (HAP) and CAP is that patients with HAP live in long-term care facilities or have recently visited a hospital. CAP is common, affecting people of all ages, and its symptoms occur as a result of oxygen-absorbing areas of the lung (alveoli) filling with fluid. This inhibits lung function, causing dyspnea, fever, chest pains and cough.

As used herein, the term "dyspnea" has its ordinary meaning known in the art. By means of further guidance, and without limitation, dyspnea is the feeling that one cannot breathe well enough. The American Thoracic Society defines it as "a subjective experience of breathing discomfort that consists of qualitatively distinct sensations that vary in intensity", and recommends evaluating dyspnea by assessing the intensity of the distinct sensations, the degree of distress involved, and its burden or impact on activities of daily living. Distinct sensations include effort/work, chest tightness, and air hunger (the feeling of not enough oxygen). Dyspnea is a normal symptom of heavy exertion but becomes pathological if it occurs in unexpected situations or light exertion. In 85% of cases it is due to asthma, pneumonia, cardiac ischemia, interstitial lung disease, congestive heart failure, chronic obstructive pulmonary disease, or psychogenic causes, such as panic disorder and anxiety. Treatment typically depends on the underlying cause. As used herein, dyspnea can be used interchangeably with shortness of breath or breathlessness. It will be understood that preferably as used herein, dyspnea is not the result of heavy physical exertion.

According to the invention, acetone and/or isoprene concentration is determined in a breath sample from a subject, such as a breath sample from a subject having dyspnea. Breath samples may be collected by any means known in the art. By means of example, and without limitation, breath samples may be collected with a ReCIVA ® breath sampler. The ReCIVA draws the breath directly onto Thermal Desorption tubes (TD-tubes). These TD-tubes can subsequently be analysed for acetone and/or isoprene concentration determination, such as by GC-MS. Advantageously, TD-tubes packed with Tenax/Carbograph (Makes international, UK) can be used. These sorbents cover a wide range of VOCs, including acetone and isoprene. The use of this sampling method has multiple advantages: (1) For GC-MS analysis pre-concentration of exhaled breath on TD-tubes is mandatory (2) The high humidity of breath causes a technical challenge, which is negated by the use of a TD-tube.

Analysis of breath samples can be performed by any means known in the art. By means of example and without limitation, analysis can be performed by gas chromatography, mass spectrometry, or coupled gas chromatography-mass spectrometry (GC-MS). GC-MS is the gold standard for separation, identification and quantification of individual VOCs. As such it allows reliably identification and quantification of all VOCs in a given breath sample, such as acetone or isoprene. Analysis of breath samples may also be performed with point-of-care devices, such as portable (breath) analyzers.

Acetone and isoprene concentrations can for instance be expressed as µg/l (i.e. µg of acetone or isoprene per liter of exhaled air). Alternatively, acetone and isoprene concentrations can be expressed as ppbV (i.e. parts per billion, volumetric). At normal atmospheric pressure (1 atm) and ambient temperature (25°C), acetone concentration expressed in µg/l can be converted to ppbV as: µg/l x 422 = ppbV. In certain embodiments, acetone concentrations as described herein and expressed as ppbV are as determined at 1 atm and 25°C. At normal atmospheric pressure (1 atm) and ambient temperature (25°C), isoprene concentration expressed in µg/l can be converted to ppbV as: µg/l x 359 = ppbV. In certain embodiments, isoprene concentrations as described herein and expressed as ppbV are as determined at 1 atm and 25°C. Conversion of values when measured at different temperature or atmospheric pressure can be performed as is known in the art.

The aspects and embodiments of the invention are further supported by the following non-limiting examples.

### EXAMPLES

### EXAMPLE 1: Exhaled breath acetone as a marker for acute heart failure in patients presenting with breathlessness

Exhaled breath contains volatile end products of systemic metabolic processes (volatile organic compounds, VOC) that can be measured and quantified.

VOCs were analysed of patients with distinctive heart failure or pulmonary disease, presenting with dyspnea in the ED with breathlessness. A total of 11 patients (mean age 75 ± 8,9 year) were enrolled. Patients with heart failure included 3 patients with left ventricular dysfunction (mean left ventricular ejection fraction 23 ± 4.2%) and two patients with right ventricular dysfunction, all in decompensated phase. Patients with pulmonary diseases consisted of 5 community acquired pneumonia (CAP) and 1 chronic obstructive pulmonary disease (COPD). Exhaled breath was sampled using a commercially available sampler device and analyzed using selected ion flow tube mass spectrometry in multiple ion monitoring mode for acetone, pentane and isoprene.

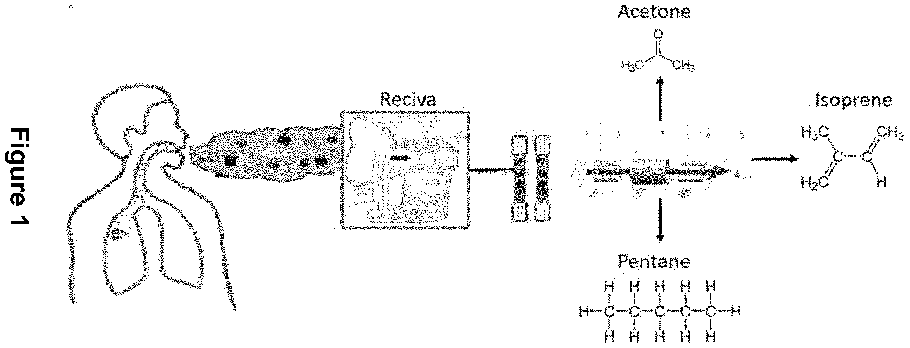

Of the measured exhaled compounds only acetone was significantly elevated in patients with heart failure (mean 2841 ± 873 ppbV, IQR 1256 ppbV) when compared to patients with lung diseases (Mean 555 ± 257 ppbV, IQR 238 ppbV), as demonstrated in Table 1 and Figure 1. Receiver operating characteristics identified an optimal cut-off value of 864 ppb of exhaled acetone to diagnose AHF with an area under the curve of 1.0. While in view of the limited number of patients screened, isoprene did not reach statistical significance (p-value > 0.05), nevertheless a clear difference between isoprene concentrations in patients with heart failure and patients with lung disease was observed.

Acetone had poor correlation with glucose (R² = -0.118) and last time of meal (R² =-0.004). Acetone did moderately correlate with NT-proBNP (R² = 0.53), although this did not research statistical significance (p = 0.4232).

**Table 1**

| VOC | Heart failure Mean (IQR) ppbV | lung disease Mean (IQR) ppbV | P-value |
|---|---|---|---|
| Isoprene | 51.1±29 (36,4) | 21 ±11,4 (11,5) | 0,082 |
| Pentane | 166±33,4 (40,2) | 174±88,7 (21,9) | 0,537 |
| Acetone | 2814±873 (1265) | 555±257 (238) | 0,004 |

The results demonstrate that acetone and/or isoprene concentration in a breath sample can be used to discriminate between heart failure and pulmonary diseases.

## Claims

1. A method for discriminating between acute heart failure and a pulmonary disease in a subject having dyspnea, comprising determining acetone and/or isoprene concentration in a breath sample from said subject.

2. Use of acetone and/or isoprene concentration in a breath sample from a subject having dyspnea as a marker for discriminating between acute heart failure and a pulmonary disease in said subject.

3. The method or use according to claim 1 or 2, wherein said pulmonary disease is COPD.

4. The method or use according to claim 1 or 2, wherein said pulmonary disease is pneumonia, preferably community acquired pneumonia.

5. The method or use according to any of claims 1 to 4, wherein acute heart failure is diagnosed in said subject if the acetone concentration is at least 700 to 900 ppbV, preferably ranging from between 700 and 900 ppbV to 10000 ppbV.

6. The method or use according to any of claims 1 to 5, wherein acute heart failure is diagnosed in said subject if the acetone concentration is at least 864 ppbV, preferably ranging from 864 ppbV to 10000 ppbV.

7. The method or use according to any of claims 1 to 6, wherein a pulmonary disease is diagnosed in said subject if the acetone concentration is less than 700 to 900 ppbV, preferably ranging from between 300 ppbV and 700 to 900 ppbV.

8. The method or use according to any of claims 1 to 7, wherein a pulmonary disease is diagnosed in said subject if the acetone concentration is less than 864 ppbV, preferably ranging from between 300 ppbV and less than 864 ppbV.

9. The method or use according to any of claims 1 to 4, wherein acute heart failure is diagnosed in said subject if the isoprne concentration is at least 25 to 35 ppbV, preferably ranging from between 25 and 35 ppbV to 200 ppbV.

10. The method or use according to any of claims 1 to 5, wherein acute heart failure is diagnosed in said subject if the isoprene concentration is at least 30 ppbV, preferably ranging from 30 ppbV to 200 ppbV.

11. The method or use according to any of claims 1 to 6, wherein a pulmonary disease is diagnosed in said subject if the isoprene concentration is less than 25 to 35 ppbV, preferably ranging from between 10 ppbV and 25 to 35 ppbV.

12. The method or use according to any of claims 1 to 7, wherein a pulmonary disease is diagnosed in said subject if the isoprene concentration is less than 30 ppbV, preferably ranging from between 10 ppbV and less than 30 ppbV.

13. The method or use according to any of claims 1 to 12, wherein said subject has left ventricular dysfunction, in particular a decreased ejection fraction and/or wherein said subject has right ventricular dysfunction, in particular a decreased ejection fraction.

14. The method or use according to any of claims 1 to 13, wherein said subject has a mean left and/or right ejection fraction of less than 50%, preferably less than 40%, more preferably less than 30%.

15. The method or use according to any of claims 1 to 14, wherein said subject is admitted to a hospital emergency department.
